# EUROPEAN PATENT APPLICATION

(11) **EP 4 632 365 A1**
(43) Date of publication of application: **15.10.2025**
(21) Application number: 25169981.5
(22) Date of filing: 11.04.2025
(51) Int. Cl.: G01N 23/223

(54) **APPARATUS AND METHOD FOR INSPECTING AN ENGINE COMPONENT**

(30) Priority: 12.04.2024 US 202463633139 P
(71) Applicant: General Electric Company, Cincinnati, Ohio 45215 (US)
(72) Inventor: DIDOMIZIO, Richard, Niskayuna, 12309 (US); DIXON III, Walter, Niskayuna, 12309 (US); HANLON, Timothy, Niskayuna, 12309 (US); OWENS, Jonathan Rutherford, Niskayuna, 12309 (US); RUSCITTO, Daniel, Niskayuna, 12309 (US); TELFEYAN, Eric John, Niskayuna, 12309 (US); KRAUSS, Michael Wylie, West Chester, 45069 (US)
(74) Representative: Openshaw & Co.

(57) **Abstract**

An apparatus and method (100) for an inspection apparatus for inspecting an engine component (12a, 12b). The inspection apparatus includes at least one controller (400) configured to receive a set of inspection parameters based on a detection metric (60). A nondestructive evaluation (NDE) instrument for scanning a predetermined area of a surface of the engine component (12a, 12b) according to the set of inspection parameters to generate a data set (70) is included. Further, the inspection apparatus includes a computer (400) configured to apply a detection algorithm (A) to the data set.

## Description

### CROSS-REFERENCE TO RELATED APPLICATIONS

This application claims priority to U.S. Provisional Application No. 63/633,139, filed on April 12, 2024, which is hereby incorporated by reference in its entirety for all purposes.

### TECHNICAL FIELD

This disclosure generally relates to an inspection apparatus and more specifically to a method for inspecting the surface of an engine component for anomalies.

### BACKGROUND

Manufactured components require inspection before and during operation. Ultrasonic (UT) evaluation, macro etch, eddy current (ECI), X-ray/computed tomography (CT), and micro-X-ray fluorescence (XRF) are all technologies employed to inspect components. Each technology has limitations that make finding anomalies on a surface of the manufactured component difficult or limited in scope. Additionally, inspection can be a slow process taking up considerable time and resources.

### BRIEF DESCRIPTION OF THE DRAWINGS

A full and enabling disclosure of the presently described technology, including the best mode thereof, directed to one of ordinary skill in the art, is set forth in the specification, which makes reference to the appended figures, in which:
FIG. 1 is a graph of a detection metric vs a target life limit illustrating a first lifing model and a second lifing model.
FIG. 2 is a schematic of processing a local data set from the local scan module.
FIG. 3 is a flow chart illustrating a method for inspecting an engine component for chemical anomalies.
FIG. 4 is a block diagram of an example programmable circuitry platform structured to execute and/or instantiate example machine-readable instructions, computing structure, and/or example operations, such as in FIGS. 1-3, to implement the inspection apparatus and associated method.

### DETAILED DESCRIPTION

Reference will now be made in detail to present embodiments of the disclosure, one or more examples of which are illustrated in the accompanying drawings. The detailed description uses numerical and letter designations to refer to features in the drawings. Like or similar designations in the drawings and description have been used to refer to like or similar parts of the disclosure.

Aspects of the disclosure generally relate to an inspection apparatus and method of inspecting an engine component, and more particularly to a micro-XRF inspection method for identifying potential chemical anomalies on the surfaces of a fielded component by way of a non-limiting example using a turbine engine component. The turbine engine component can be from either a jet engine or a land based heavy-duty gas turbine. For purposes of illustration, the present disclosure will be described with respect to inspection of the surfaces of a turbine engine component. It will be understood, however, that aspects of the disclosure described herein are not so limited and may have general applicability within other inspection methods and apparatus outside of manufactured turbine engine components for jet engines, i.e. any component for a gas turbine engine is contemplated, whether the engine component is fielded or newly manufactured.

Some known engine inspection methods have shortcomings that are overcome by the methods and apparatus disclosed and described herein. Ultrasonic (UT) evaluation, for example, lacks the ability to find some chemical anomalies of interest in some engine components because the change in grain size, or other UT-detectable feature, within the chemical anomaly compared to the parent material is not significant enough to result in a change to the sound speed that is measurable. Macroetching requires that a surface of the engine component be chemically attacked, and the analysis of that surface is dependent on both the quality of the etch and the interpretation of the final microstructure, potentially resulting in a lower probability of detection than desired. Eddy current inspection (ECI) is unable to inspect for some smaller chemical anomalies on very rough surfaces, such as peened surfaces, and relies upon the interpretation of a signal driven by the microstructure and not a direct assessment of the engine component microstructure being inspected. X-ray/Computed Tomography (CT) scanning can effectively find chemical anomalies that have a significant difference in density compared to the base metal. However, chemical anomalies with small differences in density compared to the base metal require section thicknesses that are thinner than production components, limiting its capability. Finally, historically micro-XRF inspection has been extremely time-consuming for larger components when searching for small chemical anomalies because the pixel size must be restricted to ensure enough signal is obtained within a potential chemical anomaly of interest. Additionally, typical micro-XRF systems are confined to an X/Y scan pattern with limited Z motion capabilities making them impractical for scanning complex geometry parts.

The micro-XRF inspection method and apparatus discussed herein incorporates a micro-XRF head including an X-ray source and at least one detector as part of a robot and turn table system, utilizes a computer detection algorithm for identifying possible chemical anomalies on a surface of an engine component, and performs a multi-scan inspection method to decrease the historically long inspection time.

Aspects of the disclosure provide for an inspection method and inspection apparatus that utilize the micro-XRF technique to reduce the inspection (scan) time required to find surface chemical anomalies while still meeting precision and recall requirements. While a micro-XRF inspection method will be primarily utilized, it should be understood that aspects of the disclosure herein can be applied to other processes of inspection.

"Precision," as used herein, is a measure of the number of false positives found in a population and is given by the following ratio: (True Positive)/(True Positive + False Positive).

"Recall," as used herein is a measure of the number of false negatives found in a population and is given by the following ratio: (True Positive)/(True Positive + False Negative).

"Lifing model," refers to a probabilistic lifing model which incorporates mission analysis, material properties, anomaly size and frequency distributions, part area inspected, and probability of detection of the anomaly to define a life prediction for an engine component. This model is used to set a minimum required recall level for that engine component. That is, the lifing model is used to establish a number of acceptable false negatives.

"Chemical anomaly," as used herein is a region under which the composition of one or more elements, or any combination of elements, has a statistical difference from other regions. Often, the chemical anomaly is a region that is statistically different than the expected parent alloy that is formed into the engine component. Establishing statical thresholds on variation found within the composition of elements based on a predetermined set of inspection requirements is a function of detection limits associated with the scanning instrument used and ultimately the speed under which the part can be scanned.

The term "high-speed scan" as used herein is in reference to a complete scan of an engine component surface or a predetermined portion of the engine component surface, completed as fast as possible while still meeting a predetermined set of inspection requirements and a required minimum recall level defined by the lifing model.

The term "higher resolution" as used herein can be in reference to a spatial resolution, with a smaller pixel size, or referring to an increased signal produced by a slower scan rate. The higher resolution is with respect to the resolution, the signal-to-noise ratio of the high-speed scan, or both the resolution and the signal-to-noise ratio of the high-speed scan in that the higher resolution is improved in comparison.

The term "high-resolution scan" as used herein is in reference to a complete local scan centered around a possible chemical anomaly location. The high-resolution scan has a higher resolution and is based on the set of predetermined inspection requirements. In one aspect, the high-resolution scan can be returned after the high-speed scan is complete. In another aspect, the high-resolution scan occurs solely based on the set of inspection requirements.

The term "detection metrics" as used herein describes a series of inspection bounds that must be met in order to successfully inspect an engine component. These include achieving a required precision level and recall level, finding anomalies greater than a certain defined size, and finding anomalies of a certain shape. Specific detection metrics must be defined for each type of anomaly.

The term "element maps" as used herein describes a multi-channel pixelated data set. Each pixel represents an area at a location defined in scan coordinates and a set of values that represents the integration of detector data within one or more energy windows or metadata associated with the readout occurring at the specified pixel location. Each channel represents an aggregation of detector data from one or more energy windows, which is often centered around a specific elemental characteristic X-ray emission energy peak or combinations of peaks. The energy window definitions used to create the element maps are analysis-specific and can be tuned to specific signatures associated with the chemical anomalies for which the component is being inspected. Element maps can include datasets collected via point or line scans.

The word "exemplary" may be used herein to mean "serving as an example, instance, or illustration." Any implementation described herein as "exemplary" is not necessarily to be construed as preferred or advantageous over other implementations. Additionally, unless specifically identified otherwise, all embodiments described herein should be considered exemplary.

The singular forms "a", "an", and "the" include plural references unless the context clearly dictates otherwise. Furthermore, as used herein, the term "set" or a "set" of elements can be any number of elements, including only one.

All directional references (e.g., radial, axial, proximal, distal, upper, lower, upward, downward, left, right, lateral, front, back, top, bottom, above, below, vertical, horizontal, clockwise, counterclockwise, upstream, downstream, forward, aft, etc.) are only used for identification purposes to aid the reader's understanding of the present disclosure, and do not create limitations, particularly as to the position, orientation, or use of aspects of the disclosure described herein. Connection references (e.g., attached, coupled, connected, and joined) are to be construed broadly and can include intermediate structural elements between a collection of elements and relative movement between elements unless otherwise indicated. As such, connection references do not necessarily infer that two elements are directly connected and in fixed relation to one another. The exemplary drawings are for purposes of illustration only and the dimensions, positions, order, and relative sizes reflected in the drawings attached hereto can vary.

A non-destructive evaluation (NDE) inspection system is designed to enable the inspection of a variety of engine components. The engine component can be a fielded component having been in use for a predetermined amount of time and ready for inspection. The fielded component can be an engine component, and more particularly a jet engine component. Further, the engine component can be a heavy-duty gas turbine component. Additionally, the engine component can be a new make component, billet, a forging, or a casting. The size and shape of the engine component dictates how the NDE technique is manipulated to cover the intended surface area. For example, a micro-XRF based inspection system can manipulate an X-ray source and an X-ray detector along the contours of a jet engine blade at a constant stand-off distance with the use of a robotic arm. A position of the robotic arm is coupled with an elemental spectrum obtained by the micro-XRF system to create elemental maps of the inspected surface (e.g., the surface of the jet engine blade, etc.). A single scan speed can be utilized to complete the inspection. It is also contemplated that multiple scan speeds are used in series. The different scan speeds vary the inspection parameters. The inspection parameters can be micro-XRF inspection parameters including tube excitation parameters, speed of scan, spot and pixel sizes, standoff distance, and angle of inspection constrained by part geometry limitations. A high-speed scan is used to cover larger areas at higher speeds while achieving the necessary minimum recall level. Any potential anomalies identified can be subsequently locally scanned by a high-resolution scan. The varied speed scans are used in conjunction such that they minimize or otherwise reduce an inspection time amount while meeting a required set of detection metrics.

A predetermined set of inspection requirements R can include predetermined boundaries of normal chemical variations. This set of parameters can be input by an operator or from a database, or from a combination of an operator and a database. The predetermined set of inspection requirements R can vary depending on the component being inspected. The predetermined set of inspection requirements R can include a pre-defined threshold value that is established on, by way of non-limiting example, historical data.

A detection algorithm A is a computer algorithm implemented to output information regarding the collected data sets of elemental maps. The detection algorithm A can include the ability to assess the cleanliness of the component surface in order to identify suspected chemical anomalies. For example, the detection algorithm A can be an optimization routine in which a false positive rate is experimentally determined as a function of scan condition. A combination of recall, precision, and inspection time can be experimentally produced. A recall level minimum is set by the lifing model, which then provides flexibility to balance inspection time and false positives.

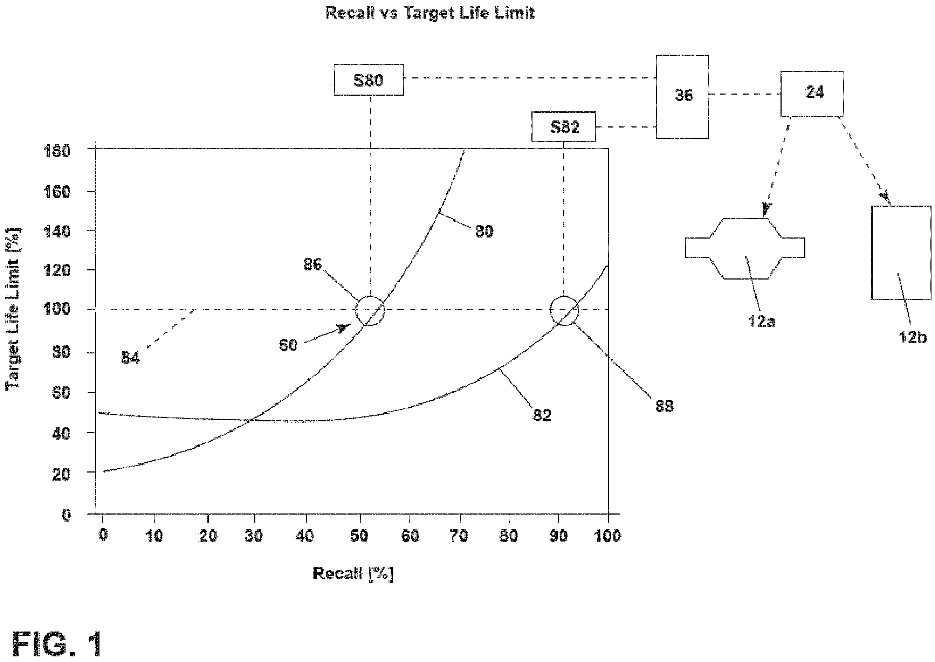

FIG. 1 is one example of a graph illustrating a detection metric vs a target life limit, where the detection metric is a recall level. The results of a first lifing model 80 and a second lifing model 82 are represented on a recall vs target life limit graph. The first lifing model 80 is for a first engine component 12a and the second lifing model 82 is for a second engine component 12b different than the first engine component 12a. Inspection parameters for the high-speed scan are selected such that they produce a validated recall level that lies within the recall level range required by the lifing model to allow the inspected engine component, by way of non-limiting example the engine components 12a, 12b described herein, to operate until its associated target lifetime. With gas turbine engines, a lifing model provides a method to assess a specific component's operating lifetime. This lifing model can be updated with inspection data collected while the part is new or with an inspection completed during service to take into account potential material anomalies. The additional information from the inspections can adjust the life prediction, increasing or decreasing the predicted lifetime of the engine component depending on the findings. In one non-limiting example, the lifing model is updated based on an inspection for an anomaly. Based on a known anomaly population, the lifing model is able to provide a range of recall level values that must be met by the inspection in order for the part to reach its targeted life. Utilizing the lifing model that incorporates the information from the known anomaly population, the high-speed scan settings are chosen such that the minimum required recall level is met for a scan on the entire surface of an engine component.

Each engine component 12a, 12b is associated with an original target life limit 84. During maintenance, a scheduled inspection, or an otherwise necessary inspection, an inspection for a possible anomaly can occur. Recall levels between 0% and 100% are possible, and, generally, the higher the recall level, the slower the scan speed. The results of the first and second lifing models 80, 82 show that there are a range of recall level values that produce life predictions beyond the target life limit 84. As a result, there is no need to inspect a part according to recall levels beyond the target life limit 84, because the part will be removed from service at the target life. The lifing models 80, 82 enable inspection parameters with lower recall levels to be utilized which, in turn, results in faster inspection times.

The first lifing model 80 intersects the 100% target life limit at a recall level of 52% while the second lifing model 82 intersects the 100% target life limit at a recall level of 90%. During inspection of the first engine component 12a according to the first lifing model 80, a first minimally acceptable recall level value 86 is 52%. During inspection of the second engine component 12b according to the second lifing model 82, a second minimally acceptable recall value 88 is 90%. Inspection parameters that yield a 52% recall level can take less time to run than the inspection parameters that yield a 90% recall level. The minimally acceptable recall level values 86, 88 translate to different sets of inspection parameters S₈₀, S₈₂ forming scanning instructions 36 for an NDE instrument 24 to scan the different engine components 12a, 12b. As such, a set of scan parameters can be selected to scan a set of anomalies, and results of the scan determine a recall level for the set of scan parameters. For example, an effectivity study including scans of a plurality of known anomalies using selected sets of scan parameters generates results that can be evaluated to determine a recall level for the set of scan parameters. The recall level can then be used with the set of scan parameters to inspect the engine component 12a, 12b.

Determining a detection metric 60 for various components, for example, the minimally acceptable recall level values 86, 88 can be completed by conducting an effectivity study (also referred to as an effectiveness study) on the inspection technique with a known set of anomalies to prove that the required recall level is met for each selected set of inspection parameters. The detection rate or recall level can be defined by the lifing model 80,82. The methods described herein can include determining the sets of inspection parameters S₈₀, S₈₂ by minimizing the required recall level along with maximizing precision and decreasing the inspection time amount within a range determined from the effectivity study and defined by the corresponding lifing models 80, 82. In one non-limiting example, the minimal recall level value is between 70% and 100%. As such, the detection metric 60 is set by input of the lifing model. For example, in addition to property data and engine operating conditions, input to the lifing model includes a value that defines a detection rate or recall level.

Similarly, the methods described herein can include further optimization. As long as the recall level is above the minimally acceptable recall level as defined by the lifing model, maximizing precision and minimizing inspection time in addition to or instead of the recall level can be pursued. The precision level value can be determined by optimizing between a number of false positives the high-speed scan returns and the amount of time it takes for the high-resolution scan to clear the false positives. The option that reduces the inspection time amount can be used as the optimized set of inspection parameters. In one non-limiting example, the optimized precision level value is between 60% and 90%.

The speed of the high-speed scan is determined such that a critical minimum recall level value is achieved, by way of non-limiting example between 70 and 100% as described herein. The critical minimum required recall level for any engine component is set by that component's corresponding lifing model. A second constraint on the maximum speed achievable by the high-speed pass is achieving a precision level, that when combined with the subsequent high resolution local scans of any potential anomalies, results in the shortest possible overall scan time. By way of non-limiting example, a precision level between 60 and 90% as described herein. The required precision level is determined via an iterative process. For example, a false positive rate can be determined as a function of scan condition. A combination of recall, precision, and inspection time can be produced. The minimum recall level is set by the lifing model, and inspection time can be balanced against false positives to satisfy the minimum recall level. The subsequent scan time of the total number of potential anomalies utilizing the high-resolution scan must be less than an incrementally slower high-speed scan speed that would produce fewer false positives.

Upon completion of the high-speed scan, the detection algorithm A is implemented. Anomaly detection is based on determining statistical deviations from baseline composition. In one example, the detection algorithm A includes a series of steps including selecting specific element maps indicative of a surface or a structural anomaly being screened for, denoising the element maps, and calculating deviations in intensity from a measure of the baseline map signal to identify a potential chemical anomaly. In one example, denoising uses a non-local means algorithm to reduce sensitivity to x-ray detector noise sources, while preserving the spatial consistency of boundary conditions, including the anomalies. The median signed deviation (MSD) is calculated for each pixel in each of the selected element maps. The MSD results are then combined via quadrature summation. The MSD algorithm is part of a family of M-estimator methods that provide a robust measure of variability in a univariate sample. A defined threshold is then applied to the distribution in the combined samples to establish how the algorithm identifies an anomaly. If a pixel or group of pixels exceed the defined threshold, then it is flagged as an anomaly, otherwise it is not and treated as expected base material.

The detection algorithm A can produce readable data in the form of a first set of element maps representing the surface of the engine component. The readable data can include a first set of element maps output from the high-speed scan. In one non-limiting example the first set of element maps can be two-dimensional. The first set of element maps can be processed to locate any possible chemical anomaly locations on the surface. In the event that a potential chemical anomaly is detected, the detection algorithm can further include marking the possible chemical anomaly location with a flag to provide a physical record of the possible chemical anomaly location. The detection algorithm A can utilize different processes for detection. In one example, the flag is based on a direct chemical assessment detected on the surface. In another example, the detection algorithm A can work by detecting differences from a baseline, by way of non-limiting example, a chemical background. In yet another example, the detection algorithm A can work by directly detecting a chemical anomaly of interest.

In one example, if the component surface meets the predetermined set of inspection requirements R associated with the presence of any chemical anomalies, then a first element map free of any flags is produced by the detection algorithm A. In this case, a signal indicating that the engine component meets the predetermined set of inspection requirements R, can be sent to an operator via, by way of non-limiting example, a computer user interface.

Some specific examples of chemical anomalies that can occur in turbine engine components are freckles, white spots, dirty white spots, stringers, oxidation, oxy-carbonitride clusters (OCNCs) large enough to act as initiating sites for failures, corrosion, and loss of coating. Freckles are chemical anomalies formed during solidification as a result of interdentritic microsegregation and fluid flow of the liquid alloy. White spots, dirty white spots, and stringers are chemical anomalies caused by the incomplete melting of material that falls into the liquid pool during the final melting step. Oxidation occurs when a metal reacts with oxygen in the atmosphere to form an oxide. Corrosion occurs when a metal reacts with contamination in the combustion process resulting in low melting compounds attacking the metal surface. A loss of coating occurs when chips or spalls in the applied coating occur, allowing the operating environment access to the underlying substrate. All these chemical anomalies result in a surface chemistry at the location of the anomaly that is statistically differentiable from the starting baseline chemistry.

At the conclusion of the high-speed scan, the detection algorithm A is run. If a suspect chemical anomaly is identified, the high-resolution scan can be run locally. At the conclusion of the high-resolution scan, a detection algorithm A is implemented again. This can be the same detection algorithm A utilized to assess the high-speed scan or a new algorithm. The detection algorithm A can be used to determine whether the suspected chemical anomaly is truly an anomaly or an operator can use the detection algorithm A output to determine whether the detection is a true positive (an anomaly) or a false positive (not an anomaly).

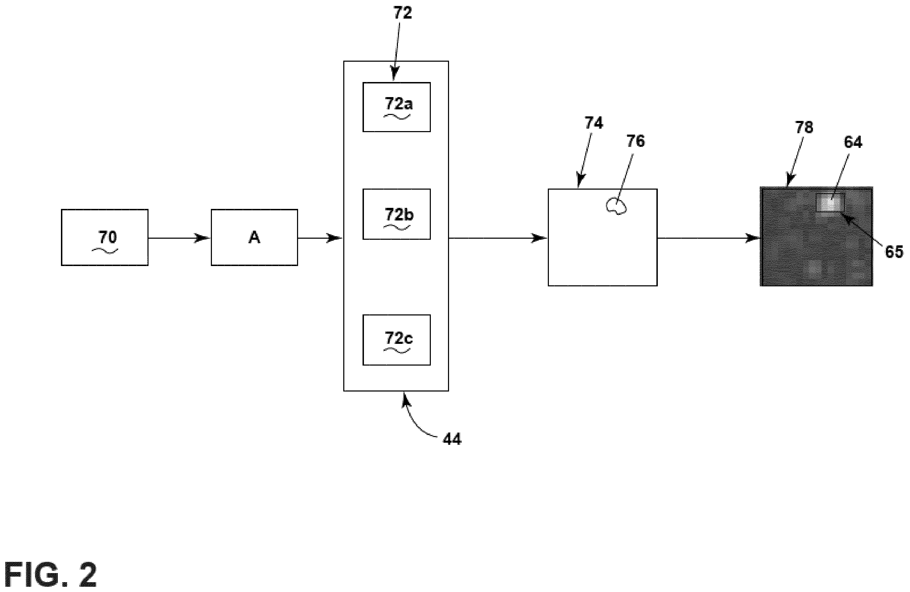

FIG. 2 illustrates an example processing of a local data set 70 using the detection algorithm A. The detection algorithm A is applied to the local data set 70 to generate a set of element maps 72, which can be readable data 44 in a non-limiting example. The set of element maps 72 can be a collection of, by way of non-limiting example, three element maps 72a, 72b, 72c, tuned to specific signatures associated with different elements. In one non-limiting example, the specific signatures are specific to metallic elements. In one aspect, an enhanced map 78, such as a de-noised map determined using a detection algorithm A and showing a potential chemical anomaly64 associated with pixel(s) 65, is derived from the set of element maps 72. Specific sets of detection metrics can be defined for each type of potential chemical anomaly and base material type.

The detection algorithm A can include determining a threshold map 74 with flag(s) 76 indicating a location at which a set of pixels 65 (the set of pixels 65 being at least one pixel) on the enhanced map 78 exceeds a pre-defined threshold value based on an as-inspected state of the surface as determined by the set of element maps 72. The as-inspected state refers to utilizing the surface to establish a total chemical distribution which is reflected in the set of element maps 72. The possible chemical anomaly location(s) 64 can be associated with the presence of a freckle, a dirty white spot, or a stringer on the surface. Further, possible chemical anomaly locations can include oxidation, corrosion, repair, OCNCs large enough to act as initiating sites for failures, loss of a coating, or a local region that deviates from the intended base metal composition as defined by the expected alloy chemistry statistical signature. A subsequent determination as to whether the flag is confirmed to be a true chemical anomaly can then be conducted.

In one non-limiting example, the threshold map 74 can be presented side-by-side with the set of element maps 72 associated with the possible chemical anomaly location. An operator or user can then make a determination regarding whether the possible chemical anomaly location matches the potential chemical anomaly or the possible chemical anomaly location is a false positive.

The high-resolution scan results in a slower inspection speed than was utilized in the high-speed scan. Limiting the area covered by the slower high-resolution scan to a predetermined area of the surface reduces the overall scan time. The higher resolution of the high-resolution scan enables a higher recall level and a higher precision level than was achieved with the high-speed scan. The higher recall level and the higher precision level of the high-resolution scan provides greater confidence in determining whether the potential anomaly identified is a true positive (a chemical anomaly of concern) or a false positive (not a chemical anomaly of concern). The detection algorithm A, as described herein, is tuned to minimize the number of false positives to achieve a precision level of between 60% and 90% and to minimize the number of false negatives to achieve a recall level of between 70% and 100%.

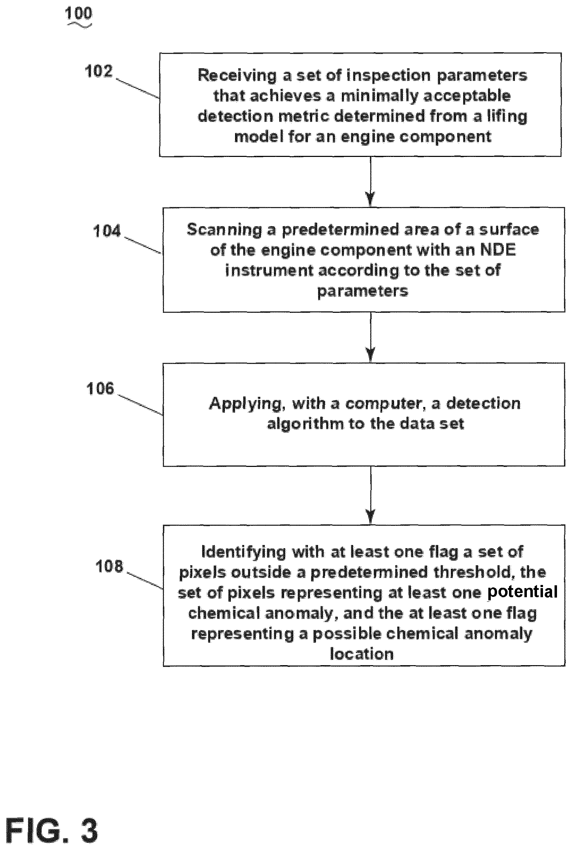

FIG. 3 is a flow chart illustrating a method 100 for inspecting an engine component (e.g., engine component 12a, 12b, etc.) as disclosed and described herein. At block 102, a set of inspection parameters is determined from the detection metric defined by a lifing model for the engine component. By way of non-limiting example, determining the set of inspection parameters S₈₀ from the lifing model 80 for the engine component includes analyzing the lifing model 80, 82 and the target life limit 84 for the engine component 12a, 12b. As shown in the example of FIG. 1, an intersection of the lifing model 80, 82 with a target life limit 84 for the respective engine component 12a, 12b provides a recall level 86, 88 for the respective engine component 12a, 12b. A set of inspection parameters S₈₀, S₈₂ can then be determined to achieve the recall level for the engine component 12a, 12b, as described herein.

At block 104, at least a portion of the surface of the engine component is scanned according to the set of inspection parameters S₈₀, S₈₂ to generate the data set, by way of non-limiting example, a global data set. For example, the NDE inspection system scans all or part of the surface of the engine component 12a, 12b at a scan speed set according to the inspection parameters S₈₀, S₈₂ (e.g., based on recall level as well as a precision level associated with the recall level, etc.). The data set includes scanned values indicative of image data, chemical composition, visual characteristics, etc.

At block 106, the detection algorithm A is applied to the data set. For example, the data set 70 generates element maps 72 from data 44 in the data set 70 and then determines a threshold map 78 based on the element maps 72. The element maps 72 can be a set of element maps 72 including, for example, three element maps 72a, 72b, 72c, tuned to specific signatures associated with different elements, such as different metallic elements.

At block 108, possible chemical anomaly location(s) based on the application of the detection algorithm A are flagged. For example, the detection algorithm A can determine a threshold map 74 including flag(s) 76 indicative of location(s) in the data 44 of the element maps 72 exceeding values in the threshold map 74. The flagged location(s) 76 (e.g., the thresholded region(s) 76) can then be further examined and/or otherwise evaluated to determine whether the anomaly is a true or false positive. If the flagged location 76 is a true positive, representing a chemical anomaly of concern, the engine component can be further inspected, repaired, removed, etc.

The detection algorithm A described herein can further include collating relevant elemental signals of interest, pre-filtering of signal noise, smoothing of input data, and statistical analysis for anomaly detection, with parameters optimized based on real scan data. In another embodiment, the detection algorithm A described herein can further include collating relevant elemental signals of interest and ground-truth information to leverage machine learning or deep learning approaches for anomaly detection.

Benefits associated with the inspection process disclosed herein include reducing the inspection time amount while still locating potential anomaly locations. The goal of an inspection process for finding potentially deleterious anomalies can be defined such that success is only achieved when all the potential anomalies are correctly identified with no false positive results. In other words, when the recall level is 100% and the precision level is 100%. As discussed herein, this definition can necessitate very time-consuming inspection parameters that either involve many repeat inspections of the same area or long duration scans meant to provide maximum resolution. While this may enable all anomalies to be found, the time required may preclude that inspection technique from being utilized in an industrial setting, resulting in scrapping parts that do not contain the potential anomaly. Including a lifing model in concert with a multi-pass scan strategy (global high-speed followed by local high-resolution) allows for a reduction in the overall inspection time amount while still ensuring that the rate of anomaly detection is great enough to ensure operation to the targeted lifetime. The reduced inspection time amount allows the inspection to be instituted such that fewer parts must be scrapped.

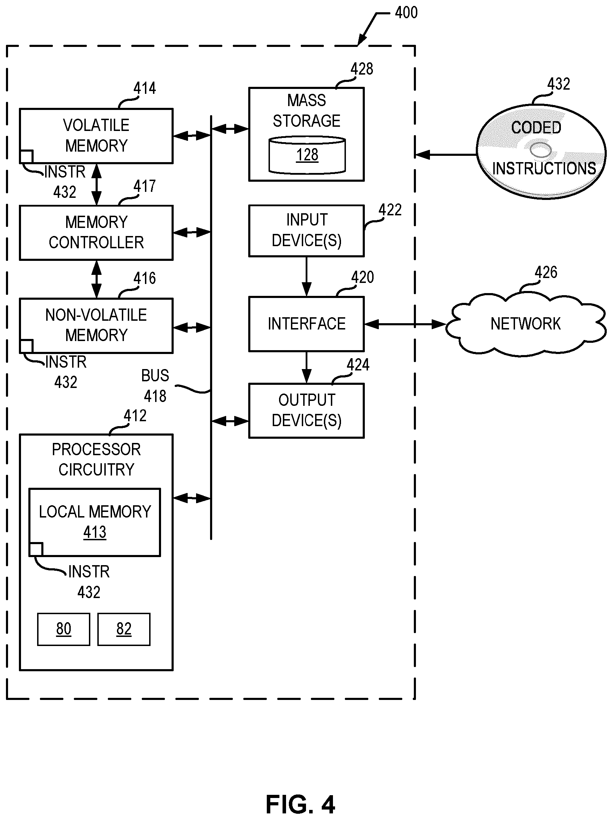

FIG. 4 is a block diagram of an example programmable circuitry platform 400 structured to execute and/or instantiate the example machine-readable instructions, computing structure, and/or example operations of FIGS. 1-3 to implement the example inspection apparatus and associated method. The programmable circuitry platform 400 can be, for example, a server, a personal computer, a workstation, a self-learning machine (e.g., a neural network), a mobile device (e.g., a cell phone, a smart phone, a tablet such as an iPad^{™}), a controller, an Internet appliance, or other computing and/or electronic device.

The programmable circuitry platform 400 of the illustrated example includes programmable circuitry 412 (also referred to as processor circuitry 412). The programmable circuitry 412 of the illustrated example is hardware. For example, the programmable circuitry 412 can be implemented by one or more integrated circuits, logic circuits, FPGAs microprocessors, CPUs, GPUs, DSPs, and/or microcontrollers from any desired family or manufacturer. The programmable circuitry 412 may be implemented by one or more semiconductor based (e.g., silicon based) devices. In this example, the programmable circuitry 412 implements the lifing model 80, 82 and/or NDE inspection system, etc.

The programmable circuitry 412 of the illustrated example includes a local memory 413 (e.g., a cache, registers, etc.). The programmable circuitry 412 of the illustrated example is in communication with a main memory including a volatile memory 414 and a non-volatile memory 416 by a bus 418. The volatile memory 414 may be implemented by Synchronous Dynamic Random Access Memory (SDRAM), Dynamic Random Access Memory (DRAM), RAMBUS^{®} Dynamic Random Access Memory (RDRAM^{®}), and/or any other type of RAM device. The non-volatile memory 416 may be implemented by flash memory and/or any other desired type of memory device. Access to the main memory 414, 416 of the illustrated example is controlled by a memory controller 417. In some examples, the memory controller 417 may be implemented by one or more integrated circuits, logic circuits, microcontrollers from any desired family or manufacturer, or any other type of circuitry to manage the flow of data going to and from the main memory 414, 416.

The programmable circuitry platform 400 of the illustrated example also includes interface circuitry 420. The interface circuitry 420 may be implemented by hardware in accordance with any type of interface standard, such as an Ethernet interface, a universal serial bus (USB) interface, a Bluetooth^{®} interface, a near field communication (NFC) interface, a Peripheral Component Interconnect (PCI) interface, and/or a Peripheral Component Interconnect Express (PCIe) interface.

In the illustrated example of FIG. 4, one or more input devices 422 are connected to the interface circuitry 420. The input device(s) 422 permit(s) a user (e.g., a human user, a machine user, etc.) to enter data and/or commands into the programmable circuitry 412. The input device(s) 422 can be implemented by, for example, an audio sensor, a microphone, a camera (still or video), a keyboard, a button, a mouse, a touchscreen, a track-pad, a trackball, an isopoint device, and/or a voice recognition system.

One or more output devices 424 are also connected to the interface circuitry 420 of the illustrated example. The output devices 424 can be implemented, for example, by display devices (e.g., a light emitting diode (LED), an organic light emitting diode (OLED), a liquid crystal display (LCD), a cathode ray tube (CRT) display, an in-place switching (IPS) display, a touchscreen, etc.), a tactile output device, a printer, and/or speaker. The interface circuitry 420 of the illustrated example, thus, typically includes a graphics driver card, a graphics driver chip, and/or graphics processor circuitry such as a GPU.

The interface circuitry 420 of the illustrated example also includes a communication device such as a transmitter, a receiver, a transceiver, a modem, a residential gateway, a wireless access point, and/or a network interface to facilitate exchange of data with external machines (e.g., computing devices of any kind) by a network 426. The communication can be by, for example, an Ethernet connection, a digital subscriber line (DSL) connection, a telephone line connection, a coaxial cable system, a satellite system, a line-of-site wireless system, a cellular telephone system, an optical connection, etc.

The programmable circuitry platform 400 of the illustrated example also includes one or more mass storage devices 428 to store software and/or data. Examples of such mass storage devices 428 include magnetic storage devices (e.g., floppy disk, drives, HDDs, etc.), optical storage devices (e.g., Blu-ray disks, CDs, DVDs, etc.), RAID systems, and/or solid-state storage discs or devices such as flash memory devices and/or SSDs.

The machine executable instructions 432, which may be implemented by the machine readable instructions of FIGS. 1-3, may be stored in the mass storage device 428, in the volatile memory 414, in the non-volatile memory 416, and/or on at least one non-transitory computer readable storage medium such as a CD or DVD which may be removable.

It should be understood that any combination of the geometry related to the inspection apparatus discussed herein is contemplated. The varying aspects of the disclosure discussed herein are for illustrative purposes and not meant to be limiting. It should be appreciated that the disclosed design can be for any applicable components, including fielded components from any engine, including a jet engine where the engine component is made of a material capable of producing chemical anomalies.

This written description uses examples to describe aspects of the disclosure described herein, including the best mode, and also to enable any person skilled in the art to practice aspects of the disclosure, including making and using any devices or systems and performing any incorporated methods. The patentable scope of aspects of the disclosure is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

Further aspects are provided by the subject matter of the following clauses:

An inspection apparatus for inspecting an engine component comprising at least one controller configured to receive a set of inspection parameters based on a detection metric determined from a lifing model for the engine component, a non-destructive evaluation (NDE) instrument for scanning a predetermined area of a surface of the engine component according to the set of inspection parameters to generate a data set, and a computer configured to apply a detection algorithm to the data set and to identify with a flag a pixel outside a pre-defined threshold, the pixel representing a potential chemical anomaly, and the flag representing a possible chemical anomaly location of the potential chemical anomaly.

The inspection apparatus of one or more preceding clauses, wherein the pixel is part of a set of pixels representing the potential chemical anomaly.

The inspection apparatus of one or more preceding clauses, wherein the NDE instrument scans the predetermined area with a high-resolution scan.

The inspection apparatus of one or more preceding clauses, wherein the predetermined area is defined by the flag.

The inspection apparatus of one or more preceding clauses, wherein the predetermined area is a first predetermined area, and wherein the NDE instrument scans a second predetermined area with a high-speed scan prior to scanning the first predetermined area with the high-resolution scan.

The inspection apparatus of one or more preceding clauses, wherein the NDE instrument is a micro-XRF instrument.

The inspection apparatus of one or more preceding clauses, wherein the detection metric is a recall level.

The inspection apparatus of one or more preceding clauses, wherein the recall level is between 70 and 100%.

The inspection apparatus of one or more preceding clauses, wherein the computer is further configured to compare the at least one flag to a known set of chemical anomaly locations during an effectivity study to establish at least one of a recall level, a precision level, or an inspection time amount.

The inspection apparatus of one or more preceding clauses, wherein the precision level is between 60 and 90%.

The inspection apparatus of one or more preceding clauses, further comprising a user interface.

A method of inspecting an engine component, the method comprising: receiving at an NDE instrument, a set of inspection parameters that achieve a detection metric determined from a lifing model for the engine component; scanning a predetermined area of a surface of the engine component with the NDE instrument according to the set of inspection parameters to generate a data set; applying, with a computer, a detection algorithm to the data set; identifying, with a flag, a set of pixels outside a pre-defined threshold, the set of pixels representing a potential chemical anomaly, and the flag representing a possible chemical anomaly location of the potential chemical anomaly; and outputting at least one of the flag, the potential chemical anomaly, or the possible chemical anomaly location.

The method of one or more of the preceding clauses, wherein receiving the set of inspection parameters comprises receiving the set of inspection parameters that achieve a minimally acceptable recall level determined from the lifing model.

The method of one or more of the preceding clauses, wherein scanning the predetermined area includes scanning with a high-speed scan setting or scanning with a high-resolution scan.

The method of one or more of the preceding clauses, wherein scanning with the high-speed scan setting returns the data set as a local data set.

The method of one or more of the preceding clauses, wherein scanning with the high-speed scan setting returns the data set as a global data set.

The method of one or more of the preceding clauses, further comprising instructing with the computer, in an event a possible anomaly is detected, the NDE instrument to scan with the high-resolution scan only locations where potential chemical anomalies are detected after scanning with the high-speed scan setting.

The method of one or more of the preceding clauses, further comprising transforming the data set into a set of element maps prior to applying the detection algorithm.

The method of one or more of the preceding clauses, further comprising denoising the set of element maps to define an enhanced map.

The method of one or more of the preceding clauses, wherein applying the detection algorithm to the data set further comprises applying the detection algorithm to the enhanced map to define a threshold map.

The method of one or more of the preceding clauses, further comprising identifying a set of pixels exceeding a threshold with the detection algorithm to define the set of flags representing the possible chemical anomaly locations.

The method of one or more of the preceding clauses, wherein the recall level is between 70 and 100%.

The method of one or more of the preceding clauses, wherein the recall level is between 50 and 100%.

The method of one or more of the preceding clauses, wherein the scanning at least a portion of a surface of the engine component comprises scanning a majority of the surface of the engine component with the high-speed scan setting.

The method of one or more of the preceding clauses, wherein determining the set of inspection parameters further comprises minimizing the recall level while still meeting a recall level required by the lifing model, maximizing a precision level, and minimizing an inspection time amount.

At least one tangible computer-readable storage medium including instructions to implement the method of any preceding clause.

At least one non-transitory computer-readable storage medium including instructions to implement the method of any preceding clause.

At least one computing device to implement the method of any preceding clause.

At least one computing device to implement the inspection apparatus of any preceding clause.

At least one non-transitory computer-readable storage medium used with the inspection apparatus of any preceding clause.

## Claims

1. An inspection apparatus for inspecting an engine component (12a, 12b), the inspection apparatus comprising:
a controller (400) configured to receive a set of inspection parameters based on a detection metric determined from a lifing model (80, 82) for the engine component (12a, 12b);
a non-destructive evaluation (NDE) instrument (400) for scanning a predetermined area of a surface of the engine component (12a, 12b) according to the set of inspection parameters to generate a data set; and
a computer (400) configured to apply a detection algorithm (A) to the data set and to identify with a flag a pixel outside a pre-defined threshold, the pixel representing a potential chemical anomaly, and the flag representing a possible chemical anomaly location of the potential chemical anomaly.

2. The inspection apparatus of claim 1, wherein the NDE instrument is to scan the predetermined area with a high-resolution scan.

3. The inspection apparatus of claim 2, wherein the predetermined area is defined by the flag.

4. The inspection apparatus of claim 3, wherein the predetermined area is a first predetermined area, and wherein the NDE instrument is to scan a second predetermined area with a high-speed scan prior to scanning the first predetermined area with the high-resolution scan.

5. The inspection apparatus of any preceding claim, wherein the NDE instrument is a micro-X-ray fluorescence (micro-XRF) instrument.

6. The inspection apparatus of any preceding claim, wherein the detection metric is a recall level.

7. The inspection apparatus of claim 6, wherein the recall level is between 70 and 100%.

8. The inspection apparatus of any preceding claim, wherein the computer is further configured to compare the flag to a known set of chemical anomaly locations during an effectivity study to establish at least one of a recall level, a precision level, or an inspection time amount.

9. The inspection apparatus of claim 8, wherein the precision level is between 60 and 90%.

10. A method (100) of inspecting an engine component, the method (100) comprising:
receiving, at a non-destructive evaluation (NDE) instrument, a set of inspection parameters that satisfy a detection metric determined from a lifing model for the engine component (102);
scanning a predetermined area of a surface of the engine component with the NDE instrument according to the set of inspection parameters to generate a data set (104);
applying, with a computer, a detection algorithm to the data set (106);
identifying, with a flag, a set of pixels outside a pre-defined threshold, the set of pixels representing a potential chemical anomaly, and the flag representing a possible chemical anomaly location of the potential chemical anomaly (108); and
outputting at least one of the flag, the potential chemical anomaly, or the possible chemical anomaly location.

11. The method of claim 10, wherein receiving the set of inspection parameters comprises receiving the set of inspection parameters that achieve a minimally acceptable recall level determined from the lifing model.

12. The method of claim 10 or 11, further including instructing with the computer, in an event a possible anomaly is detected, the NDE instrument to scan the predetermined area with a high-resolution scan only at locations where potential chemical anomalies are detected after scanning with a high-speed scan setting.

13. The method of any of claims 10 to 12, further including transforming the data set into a set of element maps prior to applying the detection algorithm.

14. The method of claim 13, further including denoising the set of element maps to define an enhanced map.

15. The method of claim 14, wherein applying the detection algorithm to the data set further comprises applying the detection algorithm to the enhanced map to define a threshold map.
